# EUROPEAN PATENT APPLICATION

(11) **EP 4 844 769 A1**
(43) Date of publication of application: **25.11.2026**
(21) Application number: 26180478.5
(22) Date of filing: 22.05.2026
(51) Int. Cl.: G16B 30/00, G16B 40/20, G06F 18/214

(54) **METHOD FOR BASE CALLING, METHOD FOR TABLE GENERATION, TABLE, RELATED APPARATUSES, AND SEQUENCING SYSTEM**

(30) Priority: 23.05.2025 CN 202510682030
(71) Applicant: GeneMind Biosciences Company Limited, Shenzhen City, Guangdong 518023 (CN)
(72) Inventor: LIU, Yurun, Shenzhen City, 518023 (CN); HUANG, Yongping, Shenzhen City, 518023 (CN); CHEN, Weiyue, Shenzhen City, 518023 (CN)
(74) Representative: Schiweck Weinzierl Koch Patentanwälte Partnerschaft mbB

(57) **Abstract**

Disclosed in the present application are a method for base calling, a method for table generation, a table, related apparatuses, and a sequencing system. In the method for base calling, all possible input features of a base calling model, that is, a plurality of corresponding groups of sequencing information, and corresponding base calling results are acquired in advance and stored in the table, such that a corresponding base calling result can be obtained by performing a query in the table according to at least one group of sequencing information of a specified cycle of sequencing, without the need to run the base calling model every time the base calling result corresponding to the specified cycle of sequencing is required, thereby improving the efficiency of acquiring the base calling result and reducing the occupation of computing resources.

## Description

### TECHNICAL FIELD

The present application relates to the field of biological information processing technologies, and in particular, to a method for base calling, a method for table generation, a table, related apparatuses, and a sequencing system.

### BACKGROUND

Next-generation gene sequencing is a high-throughput sequencing technology capable of rapidly and cost-effectively generating a large amount of sequencing data. Multiple types of data exist in a gene sequencing result file, where the multiple types of data include at least two important types of data: sequence base data (usually represented by base, and composed of A, C, G, and T) and quality score data (represented by Quality Score, and referred to as Q value for short). In current applications, a machine learning model can significantly reduce the base calling error rate by means of complex calculation and correction. The machine learning model can utilize features such as signals generated during a sequencing process as input features, and then perform prediction based on feature learning to obtain a base calling result.

The prediction process of the machine learning model has a high computational complexity, and the prediction speed of the machine learning model is relatively slow, especially under large-scale sequencing data. If the base calling result needs to be obtained via the machine learning model, the prediction and output result may not be obtained within a short time, and the occupation of computing power resources of a device will also be increased.

### SUMMARY

In view of this, the present application provides the following technical solutions:
According to a first aspect of the embodiments of the present application, a method for base calling is provided. The method includes:
acquiring a table, where the table includes a plurality of groups of sequencing information and corresponding base calling results forming key-value pairs with the respective groups of sequencing information; each of the groups of sequencing information is determined based on intensity features of signals generated by a corresponding one cycle of sequencing or a plurality of consecutive cycles of sequencing including the one cycle of sequencing; and
acquiring at least one group of sequencing information of a specified cycle of sequencing to perform an information query in the table, so as to determine a base calling result of the specified cycle of sequencing. According to a second aspect of the embodiments of the present application, a method for table generation is provided. The method includes:
   inputting each group of sequencing information among a plurality of groups of sequencing information into a base calling model, to obtain base calling results corresponding to the respective groups of sequencing information, where each of the groups of sequencing information is determined based on intensity features of signals generated by a corresponding one cycle of sequencing or a plurality of consecutive cycles of sequencing including the one cycle of sequencing; and
   storing the groups of sequencing information and the corresponding base calling results into a preset table in a form of key-value pairs, to obtain a table applied to base calling.

According to a third aspect of the embodiments of the present application, a table is provided. The table is generated based on the method for table generation according to the second aspect of the embodiments of the present application.

According to a fourth aspect of the embodiments of the present application, an apparatus for base calling is provided. The apparatus includes:
a first acquisition unit, configured to acquire a table, where the table includes a plurality of groups of sequencing information and corresponding base calling results forming key-value pairs with the respective groups of sequencing information; each of the groups of sequencing information is determined based on intensity features of signals generated by a corresponding one cycle of sequencing or a plurality of consecutive cycles of sequencing including the one cycle of sequencing; and
a query unit, configured to acquire at least one group of sequencing information of a specified cycle of sequencing to perform an information query in the table, so as to determine a base calling result of the specified cycle of sequencing.

According to a fifth aspect of the embodiments of the present application, an apparatus for table generation is provided. The apparatus includes:
a second acquisition unit, configured to input each group of sequencing information among a plurality of groups of sequencing information into a base calling model, to obtain base calling results corresponding to the respective groups of sequencing information, where each of the groups of sequencing information is determined based on intensity features of signals generated by a corresponding one cycle of sequencing or a plurality of consecutive cycles of sequencing including the one cycle of sequencing; and
a storage unit, configured to store the groups of sequencing information and the corresponding base calling results into a preset table in a form of key-value pairs, to obtain a table applied to base calling.

According to a sixth aspect of the embodiments of the present application, a sequencing system is provided.

The sequencing system includes:
a memory, configured to store an application program and a table, where the table includes a plurality of groups of sequencing information and base calling results forming key-value pairs with each of the plurality of groups of sequencing information; each of the groups of sequencing information is determined based on intensity features of signals generated by a corresponding one cycle of sequencing or a plurality of consecutive cycles of sequencing including the one cycle of sequencing; and
a processor, configured to execute the application program to implement:
   acquiring a table; and
   acquiring at least one group of sequencing information of a specified cycle of sequencing to perform an information query in the table, so as to determine a base calling result of the specified cycle of sequencing. From the above technical solutions, the present application discloses a method for base calling, a method for table generation, a table, related apparatuses, and a sequencing system. In the method for base calling, the table is acquired, and at least one group of sequencing information of a specified cycle of sequencing is acquired to perform an information query in the table, such that a base calling result of the specified cycle of sequencing is determined. The table includes a plurality of groups of sequencing information and corresponding base calling results forming key-value pairs with the respective groups of sequencing information, and the base calling results are determined based on each group of sequencing information and a base calling model. By generating the table using the groups of sequencing information and the corresponding base calling results, problems of low computational efficiency and processing complexity caused by running the base calling model to obtain the base calling result during each base calling process are solved, and the efficiency of obtaining the base calling result is improved by means of table lookup.

### BRIEF DESCRIPTION OF THE DRAWINGS

In order to more clearly illustrate the technical solutions in the embodiments of the present application, the drawings required for use in the description of the embodiments will be briefly described below. It is obvious that the drawings in the description below are only embodiments of the present application, and other drawings can be derived from the provided drawings by those of ordinary skill in the art without making inventive efforts.
FIG. 1 is a schematic flowchart of a method for base calling according to an embodiment of the present application;
FIG. 2 is a schematic partial view of a table according to an embodiment of the present application;
FIG. 3 is a comparison diagram of time rising curves according to an embodiment of the present application;
FIG. 4 is another comparison diagram of time rising curves according to an embodiment of the present application;
FIG. 5 is a schematic diagram of a table of bases and quality scores according to an embodiment of the present application;
FIG. 6 is a schematic flowchart of a method for table generation according to an embodiment of the present application;
FIG. 7 is a schematic structural diagram of an apparatus for base calling according to an embodiment of the present application; and
FIG. 8 is a schematic structural diagram of an apparatus for table generation according to an embodiment of the present application.

### DETAILED DESCRIPTION

The technical solutions in the embodiments of the present application will be clearly and completely described below with reference to the drawings in the embodiments of the present application. Apparently, the described embodiments are merely some of the embodiments of the present application instead of all of the embodiments. Based on the embodiments in the present application, all other embodiments obtained by those of ordinary skill in the art without creative efforts shall fall within the protection scope of the present application.

Terms such as "first" and "second" in the present application are used to distinguish different objects, and are not used to describe a specific order. Furthermore, the terms "comprise", "include", and "have" as well as any variations thereof, are intended to encompass a non-exclusive inclusion. For example, a process, method, system, product, or device that includes a list of procedures or units is not limited to the listed procedures or units but may include other procedures or units not listed.

In the implementations of the present application, the term "sequencing" may also be referred to as "nucleic acid sequencing" or "gene sequencing". The three are used interchangeably and refer to the determination of the type and order of bases or nucleotides (including nucleotide analogs) in a nucleic acid molecule. The sequencing involves the process of binding nucleotides to a nucleic acid template and acquiring the corresponding signals emitted by the nucleotides (including analogs). The sequencing includes sequencing by synthesis (SBS) and/or sequencing by ligation (SBL), including DNA sequencing and/or RNA sequencing.

The sequencing generally involves a plurality of cycles of processes to achieve the determination of the types and sequence of a plurality of bases or nucleotides on the nucleic acid template. The embodiments of the present application refer to each cycle of the "process to achieve the determination of the types and sequence of multiple bases or nucleotides on the nucleic acid template" as "one cycle of sequencing". "One cycle of sequencing", also known as "sequencing cycle", may be defined as the completion of one base extension of the four types of nucleotides/bases; in other words, "one cycle of sequencing" may be defined as the determination of the base or nucleotide type at any specified position on the nucleic acid template. For sequencing platforms that achieve sequencing on the basis of polymerization or ligation reactions, one cycle of sequencing includes the process of binding four types of nucleotides (including nucleotide analogs) to the nucleic acid template at a time according to the base complementary rule and acquiring the corresponding signals emitted. For platforms that achieve sequencing based on the polymerization reaction, a reaction system includes a nucleotide as a reaction substrate, a polymerase, and a nucleic acid template. A sequence fragment (a sequencing primer) binds to the nucleic acid template, and based on the base pairing rules and the principle of polymerization reaction, the nucleotide added as the reaction substrate is ligated to the sequencing primer under the catalysis of the polymerase to achieve the binding of the nucleotide to a specific position on the nucleic acid template. Generally, one cycle of sequencing may include one or more base extensions (repeats). For example, four types of nucleotides are sequentially added to the reaction system to each perform base extensions and corresponding acquisition of reaction signals, and one cycle of sequencing includes four base extensions; for another example, four types of nucleotides are added to the reaction system in any combinations (such as in pairs or in one-three combinations), the two combinations each perform base extensions and corresponding acquisition of reaction signals, and one cycle of sequencing includes two base extensions; for yet another example, four types of nucleotides are added simultaneously to the reaction system for base extension and acquisition of reaction signals, and one cycle of sequencing includes one base extension.

For example, the next-generation sequencing technology utilizes the characteristic that different fluorescent molecules have different fluorescence emission wavelengths and adopts different fluorescent molecules to label the substrates for the base extension reaction. After the base extension reaction, laser irradiates the fluorescent molecules to excite the fluorescent molecules to generate fluorescence signals. The fluorescence signals with specific wavelengths are acquired through an optical sensor, and the types of bases in the bound fluorescent molecules are identified on the basis of the fluorescence signals. Taking four-color sequencing as an example, four different fluorescent molecules are used to label four different bases (A, T/U, G, C), respectively. During one cycle of sequencing, the four bases are simultaneously added to complete one base extension reaction, laser irradiation is performed to excite the fluorescent molecules to generate fluorescence signals, and an optical imaging system is utilized to acquire the fluorescence signals generated by the different fluorescent molecules and form images. In one mode, image processing and fluorescence signal position location are performed on the images to perform base cluster detection, and template construction is performed according to base cluster detection results of a plurality of images corresponding to sequencing signal responses of different base types, so as to construct positions of all base cluster template spots. Then, according to the template, extraction of optical data (that is, mainly extraction of fluorescence signal intensities) is performed on the filtered images, and then the fluorescence signal intensities are corrected. Finally, base types are identified according to the maximum intensity at the position of each base cluster template spot. By means of a plurality of cycles of sequencing, the base sequence of the nucleic acid template can be obtained.

Multiple types of data will appear in a file containing next-generation sequencing results, where the main data includes sequence base data (represented by base: composed of A, C, G, and T/U) and quality score data (Quality Score, referred to as Q value for short). The base data, after complex calculation and correction by machine learning, has a lower error rate, and the quality score of the base data is relatively more accurate. In the process of obtaining the base calling result via the machine learning model, the calculation time for the machine learning model to predict the base calling result according to sequencing information is relatively long, and the occupied computing resources are also relatively large, thereby reducing the efficiency of predicting and outputting the base calling result. Therefore, the embodiments of the present application provide a method for base calling. The method can improve the efficiency of obtaining the base calling result and reduce the occupation of computing resources.

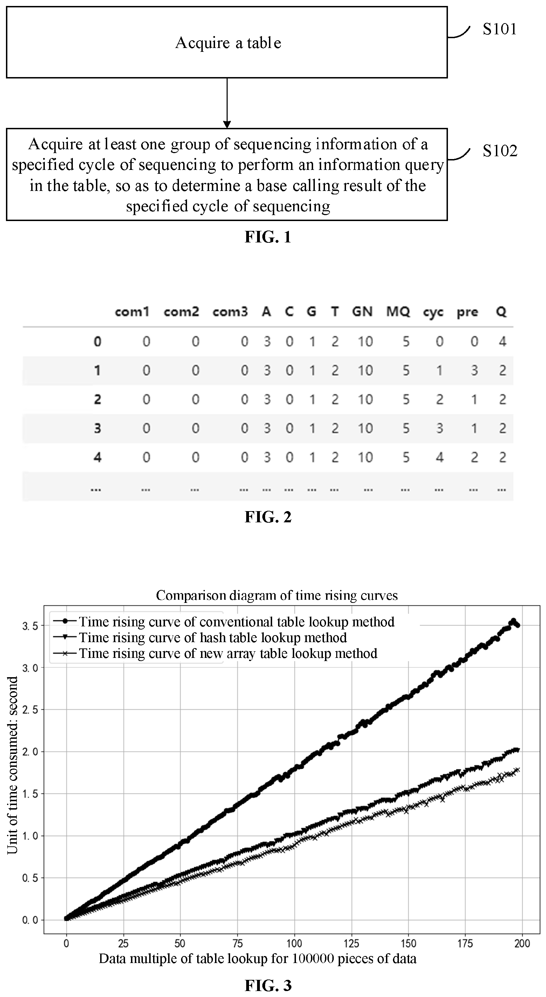

Referring to FIG. 1, FIG. 1 is a schematic flowchart of a method for base calling according to an embodiment of the present application. The method may include the following steps.

In S101, a table is acquired.

In S102, at least one group of sequencing information of a specified cycle of sequencing is acquired to perform an information query in the table, so as to determine a base calling result of the specified cycle of sequencing.

In the embodiments of the present application, the table includes a plurality of groups of sequencing information and corresponding base calling results forming key-value pairs with the respective groups of sequencing information. In other words, the table in the embodiments of the present application includes a plurality of key-value pairs, where a "key" in each key-value pair is a group of sequencing information, and a "value" is a base calling result corresponding to the group of sequencing information. Each group of sequencing information is determined based on intensity features of signals generated by a corresponding one cycle of sequencing or a plurality of consecutive cycles of sequencing including the one cycle of sequencing. The base calling results are determined based on each group of sequencing information and a base calling model. The base calling model is a machine learning model obtained by performing training based on sequencing information samples, and the base calling results output by the base calling model may include a base calling type, and may further include quality score data corresponding to a called base. Each group of sequencing information may be a signal feature that can be used to identify a corresponding base calling result, and a sequencing system can call bases by detecting a fluorescence signal, an electrical signal, or other physical signals. In the embodiments of the present application, each group of sequencing information is mainly determined according to fluorescence signal intensity features. The sequencing information of one cycle of sequencing refers to the sequencing information obtained in a single cycle of sequencing; a plurality of consecutive cycles of sequencing including the one cycle of sequencing may, for example, include a current cycle of sequencing and preceding and succeeding cycles of sequencing corresponding to the current cycle of sequencing. For example, if the current cycle is the 5th cycle of sequencing, the plurality of consecutive cycles of sequencing corresponding to the 5th cycle of sequencing may be the 3rd cycle, the 4th cycle, the 5th cycle, and the 6th cycle of sequencing. In this way, signal intensity features obtained by means of a plurality of cycles of sequencing can make calling results more accurate during the subsequent base calling process.

In the generated table used for querying to obtain a base calling result, each group of sequencing information, together with one corresponding base calling result thereof, forms a key-value pair; that is, each group of sequencing information and the one corresponding base calling result thereof are matched and stored correspondingly. For example, a first group of sequencing information corresponds to a first base calling result, a second group of sequencing information corresponds to a second base calling result, ..., and an n-th group of sequencing information corresponds to an n-th base calling result. In this way, during the subsequent table querying process, a corresponding base calling result can be found according to a current group of sequencing information without rerunning the base calling model, thereby improving the efficiency of obtaining the base calling result.

Correspondingly, during a process in which a base calling result corresponding to a specified cycle of sequencing needs to be queried, an information query can be performed in the table using a group of sequencing information corresponding to the specified cycle of sequencing to obtain a corresponding base calling result; alternatively, a query can be performed in the table using a group of sequencing information determined according to intensity features of signals generated by a plurality of consecutive cycles of sequencing including the specified cycle of sequencing to obtain a corresponding base calling result. For example, an information query can be performed in the table according to fluorescence brightness information of a current specified cycle of sequencing, to obtain a corresponding base type and a quality score corresponding to the base type.

In the embodiments of the present application, input information corresponding to different base calling models may be different. Inventors store all conceivable possible input features of the base calling models (that is, a plurality of groups of input sequencing information) and corresponding output results (that is, base calling results, which may include base calling types and corresponding quality parameters) into the table, such that a corresponding base calling result can be obtained by performing a query in the table according to at least one group of sequencing information of a specified cycle of sequencing. Thus, there is no need to run the base calling model every time the base calling result of the specified cycle of sequencing is needed, thereby improving the efficiency of acquiring the base calling result and reducing the occupation of computing resources.

The method for base calling according to the embodiments of the present application is described below in combination with corresponding application scenarios.

In the embodiments of the present application, each group of sequencing information stored in the table is determined based on intensity features of signals generated by a corresponding one cycle of sequencing or a plurality of consecutive cycles of sequencing including the one cycle of sequencing. The purpose of the present application is to accurately represent signal features in the sequencing and simultaneously reduce the space occupation of data storage in the table, such that the subsequent table query can be quickly implemented. In one implementation, the intensity features are intensity features obtained after segmentation processing is performed, according to a preset number of segments, on intensity data of the signals generated by the one cycle of sequencing or the plurality of consecutive cycles of sequencing including the one cycle of sequencing, corresponding to each group of sequencing information. The intensity data characterizes a raw intensity or a corrected intensity of a signal; the corrected intensity is an intensity after correction is performed on the raw intensity; correspondingly, the correction includes at least one of crosstalk correction, phasing/prephasing correction, or spillover correction, and the correction may also be set according to an actual application scenario requirement.

In this implementation, the raw intensity may be the intensity data of a fluorescence signal directly detected by an optical imaging system, such as an actual brightness value of the fluorescence signal detected by the optical imaging system and represented on an image. The raw intensity of the fluorescence signal is not subjected to any processing, and the raw intensity reflects an initial state of the fluorescence signal but may contain noise, interference, or other errors. To make the intensity data more accurate, the raw intensity of the fluorescence signal can be corrected to obtain a corrected intensity, so as to eliminate noise, interference, and errors in the fluorescence signal and enable the fluorescence signal to better approximate its true value, thereby improving the accuracy of base calling.

Crosstalk refers to a phenomenon in a multi-channel optical imaging system where a signal of one channel causes interference to signals of other channels. During an imaging process, especially in multi-color fluorescence imaging, different fluorescence channels may interfere with each other. The interference degree between different channels can be identified and quantified by means of crosstalk correction, and then the signal intensity of each channel is adjusted to eliminate interference from signals of other channels, thereby improving the purity and accuracy of signals, and ensuring that a signal detected by each channel only comes from the fluorescence of a corresponding wavelength and is not interfered with by signals in other channels.

Phasing/prephasing is manifested as phasing (or phase) or prephasing (or prephase). Phasing means that a nucleotide analog, supposed to react and be incorporated into a nucleic acid template in a P-th cycle of reaction, is delayed to participate in the reaction in a (P+1)-th cycle. Prephasing means that a nucleotide analog, supposed to react and be incorporated into a nucleic acid template in a P-th cycle of reaction, participates in the reaction in advance in a (P-1)-th cycle; that is, crosstalk occurs between adjacent sequencing cycles within the same channel. In this case, a correct sequencing signal for the cycle of sequencing cannot be identified; that is, sequence information of the nucleic acid template molecule cannot be accurately obtained. Therefore, such a phase error needs to be corrected, thereby improving the accuracy of base calling.

Spillover refers to the presence of a signal brightness "spillover" between adjacent base clusters, causing the brightness of the adjacent base clusters to superimpose on each other. For a base cluster signal, to eliminate such superimposed brightness, the sum of the surrounding brightness is subtracted according to a certain proportion. The specific expression for spillover correction is shown as formula (1). $Intsbleed \left(i\right) = IntsR \left(i\right) - bld ∗ {\sum }_{j ∈ {N}_{i}} IntsR \left(j\right)$ (1) where *Intsbleed*(*i*) represents a signal subjected to spillover correction; *IntsR*(*i*) represents a raw signal of a center base cluster; *bld* represents an empirical parameter; *Nᵢ* represents a set of all spatial neighboring base cluster points of an i-th base cluster; and *IntsR*(*j*) represents a signal intensity of an adjacent base cluster. By means of spillover correction, the accuracy and reliability of a signal can be improved.

The various correction processes described above are exemplified based on corresponding application scenarios; besides these processes, corresponding correction processing modes may also be determined based on actual application scenarios. For example, there may also be base bias correction, chemical correction, time drift correction, and the like. The embodiments of the present application do not limit the various correction methods, as long as the accuracy of the signal can be improved.

Intensity data, whether raw intensity or corrected intensity, generally consists of continuous floating-point values. If the floating-point values are directly stored in a table, a relatively large amount of memory will be occupied, affecting the subsequent query efficiency. In addition, if the floating-point values are used for base calling model training, the complexity of the feature values mentioned below will be increased. Therefore, in the embodiments of the present application, the intensity data is data having undergone segmentation processing according to a preset number of segments. The preset segmentation can be determined according to a data range corresponding to typical intensity data; for example, the intensity data characterizes a brightness value of a sequencing signal, and the preset segmentation refers to a total number of intervals for discretizing and segmenting the brightness value, and is used to convert continuous floating-point signals into integer features, thereby reducing the occupation of storage space. Exemplarily, if the brightness range of the signal is 1 to 1000 and the preset number of segments is 10, each segment corresponds to 100 units (1 to 100 is segment 1, 101 to 200 is segment 2, and so on). For example, signal brightness representing ACGT is (100, 200, 300, 400); after segmentation according to the preset number of segments, the converted integerized brightness may be (0, 1, 2, 3). In the embodiments of the present application, the specific number of segments is determined according to signal intensity distribution and accuracy requirements, mainly to strike a balance between feature discriminability and table-lookup memory efficiency.

In one implementation of the embodiments of the present application, each group of sequencing information includes at least one of the following parameters, or a combination thereof.
(1) A base type combination of M+N+1 cycles of sequencing determined based on a signal intensity feature of a current cycle of sequencing, signal intensity features of N preceding cycles of sequencing of the current cycle of sequencing, and signal intensity features of M succeeding cycles of sequencing of the current cycle of sequencing, where M and N are both integers greater than or equal to 0. Correspondingly, values of M and N may be determined according to actual application requirements, for example, determined according to a preset occupied memory space of a table, and may also be determined according to a preset base calling precision.

In one implementation, M and N may both take a value of 1, and in this case, each group of sequencing information includes a base type combination of three cycles of sequencing determined based on a signal intensity feature of a current cycle of sequencing, a signal intensity feature of a preceding cycle of sequencing of the current cycle of sequencing, and a signal intensity feature of a succeeding cycle of sequencing of the current cycle of sequencing.

For example, signal brightnesses of a certain base cluster template spot undergoing a current cycle (an n-th cycle) of sequencing in four A/C/G/T channels of an optical imaging system (the four A/C/G/T channels are respectively used to detect the signal on an A base, the signal on a C base, the signal on a G base, and the signal on a T base, sometimes also referred to herein as four A, T/U, C, and G base channels, or referred to as four base channels) are respectively: A = 80, C = 150, G = 200, T = 50; signal brightnesses of a preceding cycle (an (n-1)-th cycle) of sequencing in the four A/C/G/T channels are respectively A = 30, C = 180, G = 90, T = 40; and signal brightnesses of a succeeding cycle (an (n+1)-th cycle) of sequencing in the four A/C/G/T channels are respectively A = 70, C = 60, G = 210, T = 20. If a base type is determined according to the strongest brightness, a base type incorporated onto the base cluster template spot in the current cycle of sequencing is G, a base type incorporated onto the base cluster template spot in the preceding cycle of sequencing is predicted to be C, and a base type incorporated onto the base cluster template spot in the succeeding cycle of sequencing is predicted to be G; thus, a corresponding base type combination of the three cycles is CGG. The information may be used as sequencing information during table lookup.

In one implementation, M may take a value of 0, and N may take a value of 2. In this case, each group of sequencing information includes a base type combination of three cycles of sequencing determined based on a signal intensity feature of a current cycle of sequencing and signal intensity features of two preceding cycles of sequencing of the current cycle of sequencing.

It should be noted that in the embodiments of the present application, during the processing of obtaining a corresponding base calling result by using a base combination of corresponding sequencing cycles, the base combination herein is determined based on base brightness information of the corresponding sequencing cycles. For example, the base combination of the corresponding sequencing cycles is a base combination of three cycles including a current sequencing cycle, a preceding sequencing cycle, and a succeeding sequencing cycle. The base combination herein is a preliminary calling result of a current base, a preceding base, and a succeeding base. The preliminary calling result is a base type determined according to brightness information during sequencing, for example, by directly comparing the raw brightness information of the four base channels A, G, C, and T; or the preliminary calling result may be a base preliminary calling result determined according to a corrected brightness (the final brightness used after background removal, crosstalk correction, phasing/prephasing correction, brightness normalization correction, and the like). That is, when the base preliminary calling result is obtained, the base type of the current sequencing cycle is determined according to the channel with the strongest raw brightness or corrected brightness among the four base channels.

When a base calling model is used to obtain the base calling result, the base calling model can determine a final base calling result output by the base calling model based on the above base preliminary calling result. The base calling model is a machine learning model that learns from sequencing information to output the base type of the current sequencing cycle. During the training process of the base calling model, in addition to training according to the base preliminary calling result obtained based on the brightness information described above, training may also be performed according to other sequencing information, for example, some features of a sequencer, and features of a sequencing signal changing over time. In this way, multidimensional information can be used to improve the accuracy and reliability of the base calling model for base calling.

(2) A base type combination of K cycles of sequencing determined based on signal intensity features of K preceding cycles of sequencing of the current cycle of sequencing, where K is an integer greater than or equal to 1. The specific value of K may be determined according to actual scenario requirements and a preset information configuration format in the table.

In one implementation, the value of K may be 3. In this case, each group of sequencing information may be a base type combination of three cycles of sequencing determined based on signal intensity features of three preceding cycles of sequencing of the current cycle of sequencing. For example, according to signal brightness, the base type of the preceding cycle of the current cycle is obtained as A, the base type of the two preceding cycles of the current cycle is obtained as C, and the base type of the three preceding cycles of the current cycle is obtained as G; in this case, the sequencing information may be represented by GCA. In the embodiments of the present application, signal intensity features and base types of preceding and succeeding cycles may be combined to determine a base calling result of a specified sequencing cycle, thereby enhancing the robustness of prediction.

In another implementation of the embodiments of the present application, each group of sequencing information may also be intensity feature rankings of signals generated by the current cycle of sequencing in the four base channels A, T/U, C, and G. If an actual value of a signal intensity generated by each cycle of sequencing is directly stored in the table, the storage space occupied by the table is caused to be relatively large, and the query efficiency is also reduced during the subsequent table lookup process. Therefore, in the embodiments of the present application, an actual intensity feature may be converted into an intensity feature ranking (or represented as a brightness ranking). In this way, the signal intensity feature can be characterized, and the table lookup efficiency can also be improved. For example, if the brightness of ACGT corresponding to the current cycle of sequencing is (100, 200, 300, 600), a converted intensity feature ranking may be represented by (0, 1, 2, 3).

Using an intensity feature ranking to represent sequencing information can save storage space occupied by a table, but may also lose some precision in some application scenarios. For example, when intensity features representing two certain base channels are relatively close, the brightness ranking will lose a certain degree of precision. To solve this problem, in one implementation of the embodiments of the present application, a first target parameter of each group of sequencing information may be determined based on a maximum signal intensity value of a current cycle of sequencing, a first parameter, and a preset number of segments, where the first parameter characterizes the sum of the maximum signal intensity value of the current cycle of sequencing and a second maximum signal intensity value of the current cycle of sequencing. In this implementation, the maximum signal intensity value and the second maximum signal intensity value of the current cycle of sequencing may be determined based on intensity feature rankings of signals generated by the current cycle of sequencing in the four base channels A, T/U, C, and G. Specifically, when the first parameter is calculated, the maximum signal intensity value of the current cycle of sequencing and the second maximum signal intensity value of the current cycle of sequencing may be calculated according to an actual signal intensity value at the position of each base cluster template spot. Exemplarily, during the current cycle of sequencing, if intensities of signals generated at the position of a certain base cluster template spot in the four base channels A, T/U, C, and G are respectively (70, 15, 10, 5), the maximum signal intensity value generated at the position of the base cluster template spot is 70, the second maximum value is 15, and the sum of the signal intensities of the four base channels is 100. The preset number of segments refers to converting discretized data into a finite integer to facilitate the rapid matching in a table lookup method. The preset number of segments (W) is the total number of intervals into which continuous values are segmented, for example, W = 10. If the first target parameter is represented by GN, then GN = [the maximum signal intensity value of the current cycle of sequencing / (the maximum signal intensity value of the current cycle of sequencing + the second maximum signal intensity value of the current cycle of sequencing)] * W. In this way, the relationship between the maximum signal intensity value and the second maximum signal intensity value can be obtained based on the first target parameter, which can make up for the precision loss caused by representing the signal intensity feature using the intensity feature ranking.

In another implementation of the embodiments of the present application, each group of sequencing information may further include a second target parameter determined based on a maximum signal intensity value of the current cycle of sequencing, a second parameter value, and a preset number of segments. The second parameter characterizes the sum of signal intensities of the four base channels of the current cycle of sequencing. The second target parameter may characterize the magnitude relationship between the maximum signal intensity value and the total signal intensity of the current cycle of sequencing, which can further make up for the precision loss caused by representing the signal intensity feature using the intensity feature ranking. For example, if the preset number of segments is W, and the second target parameter is represented by MQ, then MQ = (the maximum signal intensity value of the current cycle of sequencing / the sum of the signal intensities of the four base channels of the current cycle of sequencing) * W.

It should be noted that the sequencing information described above is illustrated by taking corresponding implementations as examples, and the corresponding sequencing information may also be a corresponding combination of data determined in the various implementations described above. For example, each group of sequencing information may include: intensity feature rankings of signals generated by the current cycle of sequencing in four base channels A, T/U, C, and G; and the first target parameter and the second target parameter described above. For another example, each group of sequencing information may further include: the intensity feature rankings of the signals generated by the current cycle of sequencing in the four base channels A, T/U, C, and G; the first target parameter and the second target parameter described above; and a base type combination of three cycles of sequencing determined based on a signal intensity feature of the current cycle of sequencing and signal intensity features of two preceding cycles of sequencing of the current cycle of sequencing.

Further, the intensity feature rankings of the signals generated by the current cycle of sequencing in the four base channels A, T/U, C, and G described in the various implementations are determined based on sorting, according to intensity magnitudes, raw intensities or corrected intensities of the signals generated by the current cycle of sequencing in the four base channels A, T/U, C, and G. In the embodiments of the present application, intensity features of the four base channels may be directly ranked based on the raw intensities, such that ranking information of the four base channels can be obtained quickly; or the intensity features of the four base channels may be ranked based on the corrected intensities, such that the ranking information of the four base channels can be obtained more accurately. Correspondingly, a processing method for the ranking information may be determined according to an actual application scenario requirement, and the present application does not limit the processing method for the ranking information. In one implementation of the embodiments of the present application, the plurality of groups of sequencing information are determined according to intensity features of signals generated by a plurality of cycles of sequencing, and the plurality of cycles of sequencing are divided into a plurality of segments; each segment of the sequencing includes a preset number of cycles of sequencing, and the preset number of cycles of sequencing within the same segment correspond to the same group of sequencing information.

During the process of querying a base calling result based on a table, a plurality of cycles of sequencing are divided into several segments, each segment includes a preset number of cycles of sequencing, and all cycles within the same segment share the same group of sequencing information, which can reduce the complexity of table lookup while maintaining the accuracy of base calling. For example, a sequencer continuously outputs signal intensities of a plurality of cycles of sequencing; for example, the sequencer may output signal intensities of 100 cycles of sequencing. If each segment includes 25 cycles, the 100 cycles may be divided into 4 segments. Segment 1 corresponds to cycles 1 to 25; segment 2 corresponds to cycles 26 to 50; segment 3 corresponds to cycles 51 to 75; and segment 4 corresponds to cycles 76 to 100. Then, intensity features within each segment may be extracted; for example, the intensity features include a brightness ranking, a first target parameter GN characterizing the magnitude relationship between a maximum signal intensity and a second maximum signal intensity, a second target parameter MQ characterizing the magnitude relationship between the maximum signal intensity and a total signal intensity, and a base type of a preceding sequencing cycle (which, for example, may also be a base type combination of three cycles of sequencing including a current sequencing cycle, a preceding sequencing cycle, and a succeeding sequencing cycle). The above features are combined into a unique identifier (key) of the segment; for example, the sequencing information of the segment may be represented as: ranking(0,1,2,3)_GN_MQ_base type C of the preceding sequencing cycle. Then, the sequencing information may be queried in the table to obtain the base calling result, for example, including a corresponding base calling type and a Q value.

Further, during the process of generating a table, data processing may be performed on each group of stored sequencing information and base calling results, thereby reducing the data storage occupation while improving the query efficiency in the querying process. Therefore, in the embodiments of the present application, data formatting processing may also be performed on information such as the sequencing information and the base calling result, thereby reducing the data storage occupation. Correspondingly, each group of sequencing information, the base calling results, and the preset number of cycles in the table are represented in a data format in integer units, and the data format includes at least one of a data format of a target number of bits, a type data format occupying target bytes, or a binary number format. In this implementation, to maximize the memory efficiency and table lookup speed, the sequencing information, the base calling result, and the preset number of cycles are all converted into a compact integer format for storage.

Bits of the signal brightness of a current cycle of sequencing may be truncated as needed. For example, all data may be converted into an 8-bit data format, or may be converted into a 4-bit data format. For example, signal brightness data is converted to A = 00, C = 01, G = 10, T = 11 (2 bits), thereby replacing 1 byte with 2 bits. Alternatively, a minimum byte type, such as int8 or uint8, may be used to store a value. For example, if a GN value is 0.75, the GN value may be stored as 7, thereby avoiding floating-point number occupation. Alternatively, a plurality of features may be concatenated into a binary string to serve as a data index. For example, the sequencing information includes a brightness ranking and a GN value; the brightness ranking (01) + GN (0110) = 010110, and 010110 is directly used as a memory address offset to save storage space. Specifically, taking the brightness ranking as an example, a raw brightness value may be a floating-point intensity value such as A = 80, T = 50, C = 150, and G = 200; the raw brightness value is converted into an integer from 0 to 3 by sorting according to intensity. In this case, the data storage format is uint8, which can reduce the storage occupation. For the GN value or an MQ value, if GN = 0.57, the GN value may be multiplied by a preset number of segments N and then rounded off during conversion (for example, N = 10, and in this case, GN = 0.57 × 10 ≈ 6). In this case, the data storage format is int8 with a range of -128 to 127, which is sufficient to cover segments with N ≤ 100. For the base calling result, 2 bits may be used to identify one base type, such as A: 00, C: 01, G: 10, and T: 11, and when a prediction result is G, the prediction result may be 10. Floating-point data of a Q value may be converted into an integer. The preset number of cycles is also represented by an integer. For example, the number of sequencing cycles is 1 to 100 cycles, the cycles are stored in segments with every 25 cycles being one segment, and a segment number is represented by uint8 (for example, 0 to 4).

In the embodiments of the present application, a method for constructing the base calling model includes: acquiring a training sample set; and performing machine learning modeling on each sample in the training sample set based on a specific model structure to obtain the base calling model. Each training sample in the training sample set is annotated with a feature value and a target value; the feature value is one group of sequencing information in the table, and the target value is a base calling result corresponding to the one group of sequencing information. The base calling result may include a base calling type and/or a base sequencing quality score.

A data source of the training sample set may be raw sequencing data generated using a fixed version of a sequencer, reagents, and base calling software; for example, the raw sequencing data is fluorescence signal intensity data. To improve the accuracy of the base calling model, the training sample set may cover diversified sequencing scenarios, such as high/low complexity sequences and different sequencing depths. The feature value is an input value of the base calling model, and may include: a brightness ranking of each cycle of sequencing, such as a ranking of intensities of the four channels A/T/C/G (e.g., [T(0), A(1), C(2), G(3)]); GN/MQ values, which are discretized ratios (e.g., GN = 6, MQ = 5); and a context feature, which is a base type combination of preceding and succeeding cycles (e.g., a preceding base C + a current base G + a succeeding base G). The target value is a label of the training sample, and may include: a base calling type, which is a true base (A/C/G/T) annotated by means of high-precision calibration (such as Sanger sequencing or a consensus sequence); and a Q value, which may be a quality score calculated based on an error rate.

The specific model structure may include a LightGBM model structure applicable to structured numerical features. The output includes four probability values (corresponding to A/C/G/T), and the highest probability is the predicted base. The model structure may also be a convolutional neural network (CNN) applicable to capturing local signal patterns, such as a spatial correlation of a brightness ranking. A hybrid model, such as CNN + LightGBM, may also be adopted, using a CNN to process a raw signal intensity image, concatenating the CNN output with structured features such as the GN/MQ values, and inputting the concatenated result into LightGBM. Then, a gap between a predicted value and a true value of the base calling model may be calculated according to a loss function, thereby adjusting model parameters of the base calling model to obtain a final base calling model.

To facilitate data querying, in the embodiments of the present application, a plurality of sub-tables may be generated according to the number of sequencing cycles, so as to perform querying in a corresponding sub-table according to the current number of sequencing cycles, thereby improving the query efficiency. Correspondingly, acquiring the at least one group of sequencing information of the specified cycle of sequencing to perform the information query in the table, so as to determine the base calling result of the specified cycle of sequencing includes:
determining, in the case where the table includes at least one sub-table, a cycle number range corresponding to the specified cycle of sequencing; determining a target sub-table in the table based on the cycle number range; and performing the information query in the target sub-table based on the at least one group of sequencing information of the specified cycle of sequencing to determine the base calling result of the specified cycle of sequencing.

Each sub-table in the table has recorded information of a corresponding cycle number range, the recorded information characterizes a plurality of groups of sequencing information under the cycle number range and base calling results forming key-value pairs with each group of sequencing information, and the cycle number range includes a preset number of cycles of sequencing. For example, data of sequencing of cycles 1 to 100 are recorded into a sub-table 1, data of sequencing of cycles 101 to 200 are recorded into a sub-table 2, and data of sequencing of cycles 201 to 300 are recorded into a sub-table 3. If the current specified cycle is 150, performing a data query in the sub-table 2 can improve the data query efficiency.

The method for base calling in the embodiments of the present application will be described below with the example of an actual application scenario.

Because signal intensity features used for training the base calling model have no fixed pattern, if sequencing information related to all intensity features is to be summarized relying on combining each group of sequencing information into a unique identification ID (equivalent to an identification code, or a Key), types of sequencing information covered by the unique identification ID are numerous (such as signal intensity information, a base type combination of a corresponding sequencing cycle, and related target parameters), and a memory of an electronic device cannot store the summarized sequencing information. Therefore, continuously changing floating-point values characterizing the signal intensities need to be subjected to segmentation processing, thereby minimizing the size of the table and reducing the complexity of the intensity features. The inventors sort signal brightnesses at the position of an arbitrary base cluster template spot on an image and assign a new brightness ranking representing a signal intensity to each brightness based on a sorted ranking. For example, the brightnesses in the four base channels A/C/G/T of a signal generated at the position of a certain base cluster template spot obtained in a certain cycle of sequencing are (100, 200, 300, 600); then, a converted brightness ranking is (0, 1, 2, 3). At this point, the brightness feature of a brightness arrangement order can be obtained. Exemplarily, the magnitude relationship between the maximum brightness and the second maximum brightness of the signal at the position of the certain base cluster template spot in the four base channels may be represented by the first target parameter GN, and GN = [the maximum signal brightness at the position in the current cycle of sequencing / (the maximum signal brightness at the position in the current cycle of sequencing + the second maximum signal brightness at the position in the current cycle of sequencing)] * W, where W is a preset number of segments. The magnitude relationship between the maximum brightness of the signal at the position and the total brightness of the signal at the position in the four base channels is represented by MQ, and MQ = (the maximum brightness of the signal at the position in the current cycle of sequencing / the sum of brightnesses of the signal at the position in the four base channels in the current cycle of sequencing) * W, where W is the preset number of segments. Then, the above features are rounded off to the nearest integers, thereby obtaining a GN value in an interval with a minimum value of n1 and a maximum value of n2; then, an MQ value in an interval with a minimum value of n3 and a maximum value of n4 is obtained. The brightness feature of the brightness rankings of the four bases at the position in the preceding cycle of sequencing of the current cycle of sequencing, the brightness feature of the brightness rankings of the four bases at the position in the current cycle of sequencing, the brightness feature of the brightness rankings of the four bases at the position in the succeeding cycle of sequencing, and the sequence feature of a continuous base combination formed by the five preceding base types at the position in the current cycle of sequencing and the base type at the position in the succeeding cycle of sequencing can serve as one group of sequencing information.

A machine learning model (i.e., a base calling model, hereinafter referred to as "model 1") is trained using the segmented signal intensity data, and then model 1 is used to predict the result of each segmented combination for each group of sequencing information possibly composed of all possible segmented signal intensity data (e.g., raw intensity data may be segmented according to the intensity ranking), and the segmented combinations and predicted results are integrated into one table. Then, model 1 is used to predict and obtain the corresponding probabilities of four bases, and Q values corresponding to the bases are calculated using a method for calculating a Q value using the probability values of the four bases obtained by machine learning. Features of each group of possible sequencing information are combined to serve as an ID of a hash table, and then a machine learning predicted base corresponding to a parameter and a Q value corresponding to the base calculated from a machine learning predicted probability are placed under the corresponding hash table ID to form a key-value pair, thereby obtaining the information data of all tables.

Because an excessively large data volume of a machine learning prediction table and an excessively large data volume of input features can cause the problem of memory explosion during table lookup, a data structure for the table lookup is transformed. First, the predicted bases (which are segmented string features) are converted into numbers to reduce the memory occupation by the strings, and meanwhile, the data structure of the data is transformed. All data formats are converted into the int8 type (int8 represents a data format, and such a data format is the smallest data format in integer units in the computer storage), and char (char is also a counting unit, and like int8, is the smallest data unit format in the computer storage) is used for counting. The method can minimize the size of the memory occupied by data stored in an operating memory during prediction and reduce the size of the memory occupied by the table; in addition, the method can also reduce the complexity of the Key (a corresponding hash table ID) required for table lookup and accelerate the table lookup speed. The time complexity required for table lookup is also optimized.

Due to limited CPU read time and memory, segmented optimization is also performed on the size of the read-in table. The segmentation method divides sequencing cycles into segments based on the execution time of the sequencing. For example, 100 cycles of sequencing are divided into four segments in total, such as segment 1, segment 2, segment 3, and segment 4, with 25 cycles serving as one node for each segment. Exemplarily, if the sequencing proceeds to the 5th cycle of sequencing, the 5th cycle of sequencing belongs to segment 1; if the sequencing proceeds to the 50th cycle of sequencing, the 50th cycle of sequencing belongs to segment 2. In this way, a complex table with a length of 100 cycles can be divided into four smaller tables, thereby effectively reducing the table complexity and accelerating the table lookup speed. In addition, the occurrence of error reports caused by repeated reading and usage of IDs can also be reduced. Referring to FIG. 2, FIG. 2 illustrates a portion of the table. In the table shown in FIG. 2, com may represent a base type combination of a corresponding sequencing cycle. For example, com1, com2, and com3 may represent a base type combination of a plurality of sequencing cycles of a first form, a base type combination of a plurality of sequencing cycles of a second form, and a base type combination of a plurality of sequencing cycles of a third form. It should be noted that the number of sequencing cycles corresponding to each com may be the same or different, and base type combinations corresponding to different sequencing cycles having the same number of sequencing cycles may be selected. Specifically, for example, com1 represents the combination of the base type of the current cycle of sequencing, the base type of the preceding cycle of sequencing of the current cycle of sequencing, and the base type of the succeeding cycle of sequencing of the current cycle of sequencing; com2 represents the combination of the base type of the current cycle of sequencing and base types of the two preceding cycles of sequencing of the current cycle of sequencing; and com3 represents the base type combination of the three preceding cycles of sequencing of the current cycle of sequencing. The data corresponding to A, C, G, and T are represented according to brightness rankings. GN and MQ are represented by integers; cyc represents the number of sequencing cycles; pre represents a base type identifier in a base calling result; for example, 0 may represent base A; Q represents a quality score of a corresponding base type.

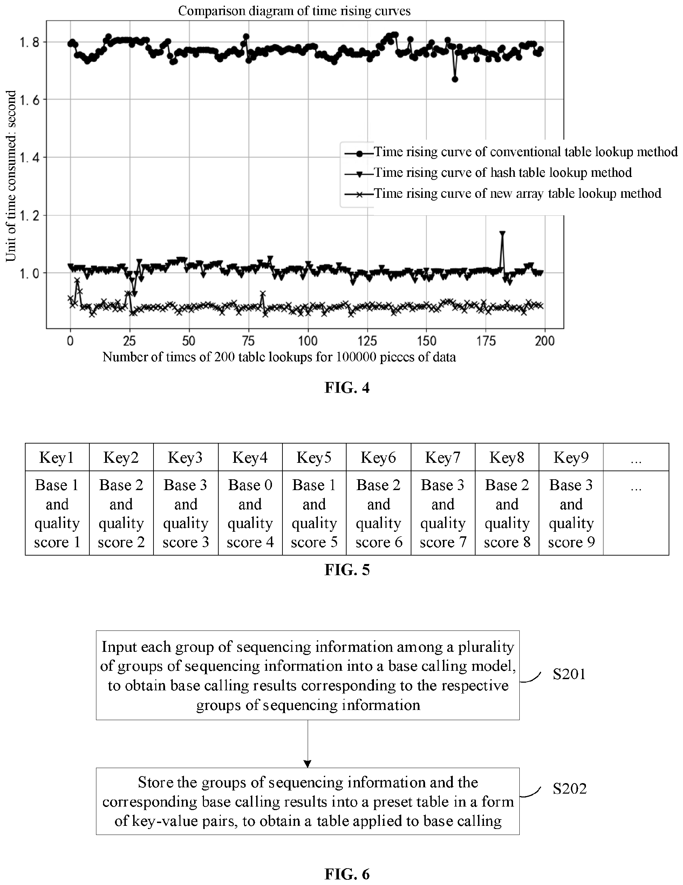

To further reduce the data storage occupation of the table and improve the table query efficiency, all char features of the table can be calculated and converted into a unique key binary number X1, and the corresponding base calling type and Q value are also converted into a corresponding unique value Y1. A synthesis method for the binary number X1 is to convert the number of bytes occupied by each feature in units of char used in a value range of the feature into a binary number X1 represented in order. Then, this X1 can be used as an array index and a mark. Subsequently, the unique value Y1 corresponding to the base and the Q value serves as the array content. In this way, the memory usage can be greatly reduced, and the table lookup speed can be accelerated. With respect to the selection of arrays, tests show that a vector array in a C++ standard library has a faster table lookup speed than a hash container (unordered_map, map) in the standard library; because although looking up a hash table also has an O(1) time complexity, the hash address corresponding to the feature needs to be calculated for each table lookup, whereas using the feature as the array index does not require this operation. Referring to FIGs. 3 and 4, in FIGs. 3 and 4, a new array table lookup method characterizes the table query method in the embodiments of the present application, and higher efficiency can be obtained for the table query method adopted in the embodiments of the present application.

According to the research on the data format of the table, brightnesses can be sorted using 0, 1, 2, and 3 in order of magnitude to sort four brightnesses. Compared to the true base brightness, this data sorting method is relatively simplified without affecting the degree of data meaning. With respect to the precision loss of the magnitude among base brightness data, the table uses the ratio of the maximum brightness to the sum of the maximum brightness and the second maximum brightness to determine the subtle difference between the maximum brightness and the second maximum brightness, thereby making up for the precision loss caused by representing the base brightness using sorting. The prominence difference in brightness is calculated by dividing the maximum brightness of a currently tested base by the total sum of the base brightness of the currently tested base, to further make up for the error caused by the precision loss in brightness. Prediction results can be recorded at the end of the table. Numbers 0, 1, 2, and 3 are used to represent the four bases A, C, G, and T, and then a corresponding Q value is calculated according to a predicted result feature of each ID. After the Q value is obtained, a new table will be used again to obtain the result data and the Q value of brightness, and then, using a re-alignment result, the true error rate is used to fine-tune and correct the machine learning Q value, thereby obtaining a more accurate Q value. A fine-tuning method is as follows: all Q values can be represented by continuous numbers; subsequently, a true error rate of the Q value under such an ID is counted; and then a true Q value is calculated based on the error rate. Then, the latest Q value is compared with the Q value calculated by machine learning to obtain an average difference, and then a difference compensation operation is performed to obtain a more accurate Q value.

According to the above steps, a highly compressed two-dimensional array table can be obtained; operations on the array yield results very quickly, and a table from which a corresponding base and a quality score can be simultaneously output is shown in FIG. 5. Using the table lookup method is equivalent to greatly accelerating the machine learning prediction speed and reducing the difficulty of machine learning engineering.

In the embodiments of the present application, a method for table generation is further provided. Related content of the method for table generation has been described in the aforementioned method for base calling, and details are not described herein again. Referring to FIG. 6, the method for table generation may include the following steps.

In S201, each group of sequencing information among a plurality of groups of sequencing information is input into a base calling model to obtain base calling results corresponding to each group of sequencing information.

Each group of sequencing information is determined based on intensity features of signals generated by a corresponding one cycle of sequencing or a plurality of consecutive cycles of sequencing including the one cycle of sequencing.

In S202, each group of sequencing information and the corresponding base calling results are stored into a preset table in a form of key-value pairs, to obtain a table applied to base calling.

Optionally, the intensity features are intensity features obtained after segmentation processing is performed, according to a preset number of segments, on intensity data of the signals generated by the one cycle of sequencing or the plurality of consecutive cycles of sequencing including the one cycle of sequencing, corresponding to each group of sequencing information. The intensity data characterizes a raw intensity or a corrected intensity of a signal; the corrected intensity is an intensity after correction is performed on the raw intensity; optionally, the correction includes at least one of crosstalk correction, phasing/prephasing correction, or spillover correction.

Optionally, each group of sequencing information includes at least one of the following parameters, or a combination thereof:
a base type combination of M+N+1 cycles of sequencing determined based on a signal intensity feature of a current cycle of sequencing, signal intensity features of N preceding cycles of sequencing of the current cycle of sequencing, and signal intensity features of M succeeding cycles of sequencing of the current cycle of sequencing,
where M and N are both integers greater than or equal to 0;
a base type combination of K cycles of sequencing determined based on signal intensity features of K preceding cycles of sequencing of the current cycle of sequencing,
where K is an integer greater than or equal to 1;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, where the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, where the second parameter characterizes a sum of signal intensities of the four base channels of the current cycle of sequencing.

Optionally, each group of sequencing information includes at least one of the following parameters, or a combination thereof:
a base type combination of three cycles of sequencing determined based on a signal intensity feature of a current cycle of sequencing, a signal intensity feature of a preceding cycle of sequencing of the current cycle of sequencing, and a signal intensity feature of a succeeding cycle of sequencing of the current cycle of sequencing;
a base type combination of three cycles of sequencing determined based on the signal intensity feature of the current cycle of sequencing and signal intensity features of two preceding cycles of sequencing of the current cycle of sequencing;
a base type combination of three cycles of sequencing determined based on signal intensity features of three preceding cycles of sequencing of the current cycle of sequencing;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, where the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, where the second parameter characterizes a sum of signal intensities of the four base channels of the current cycle of sequencing.

Optionally, the intensity feature rankings of the signals generated by the current cycle of sequencing in the four base channels A, T/U, C, and G are determined based on sorting, according to intensity magnitudes, raw intensities or corrected intensities of the signals generated by the current cycle of sequencing in the four base channels A, T/U, C, and G.

Optionally, the plurality of groups of sequencing information are determined according to intensity features of signals generated by a plurality of cycles of sequencing, and the plurality of cycles of sequencing are divided into a plurality of segments; each segment of the sequencing includes a preset number of cycles of sequencing, and the preset number of cycles of sequencing within the same segment correspond to the same group of sequencing information.

Optionally, each group of sequencing information, the base calling results, and the preset number of cycles in the table are represented in a data format in integer units, and the data format includes at least one of a data format of a target number of bits, a type data format occupying target bytes, or a binary number format. Optionally, the base calling results include base calling types and/or base sequencing quality scores. Optionally, a method for constructing the base calling model includes:
acquiring a training sample set, where each training sample in the training sample set is annotated with a feature value and a target value; the feature value is one group of sequencing information in the table, and the target value is a base calling result corresponding to the one group of sequencing information; and
performing machine learning modeling on each sample in the training sample set based on a specific model structure to obtain the base calling model.

Optionally, the specific model structure includes a LightGBM model structure.

Correspondingly, in the embodiments of the present application, a table is further provided. The table is generated based on the above method for table generation.

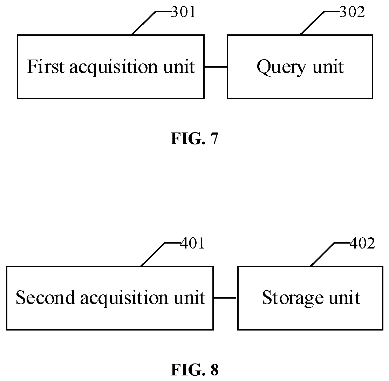

In the embodiments of the present application, an apparatus for base calling is further provided. Referring to FIG. 7, the apparatus for base calling includes:
a first acquisition unit 301, configured to acquire a table, where the table includes a plurality of groups of sequencing information and base calling results forming key-value pairs with each group of sequencing information; each group of sequencing information is determined based on intensity features of signals generated by a corresponding one cycle of sequencing or a plurality of consecutive cycles of sequencing including the one cycle of sequencing; and
a query unit 302, configured to acquire at least one group of sequencing information of a specified cycle of sequencing to perform an information query in the table, so as to determine a base calling result of the specified cycle of sequencing.

Optionally, the intensity features are intensity features obtained after segmentation processing is performed, according to a preset number of segments, on intensity data of the signals generated by the one cycle of sequencing or the plurality of consecutive cycles of sequencing including the one cycle of sequencing, corresponding to each group of sequencing information. The intensity data characterizes a raw intensity or a corrected intensity of a signal; the corrected intensity is an intensity after correction is performed on the raw intensity; optionally, the correction includes at least one of crosstalk correction, phasing/prephasing correction, or spillover correction.

Optionally, each group of sequencing information includes at least one of the following parameters, or a combination thereof:
a base type combination of M+N+1 cycles of sequencing determined based on a signal intensity feature of a current cycle of sequencing, signal intensity features of N preceding cycles of sequencing of the current cycle of sequencing, and signal intensity features of M succeeding cycles of sequencing of the current cycle of sequencing,
where M and N are both integers greater than or equal to 0;
a base type combination of K cycles of sequencing determined based on signal intensity features of K preceding cycles of sequencing of the current cycle of sequencing,
where K is an integer greater than or equal to 1;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, where the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, where the second parameter characterizes a sum of signal intensities of the four base channels of the current cycle of sequencing.

Optionally, each group of sequencing information includes at least one of the following parameters, or a combination thereof:
a base type combination of three cycles of sequencing determined based on a signal intensity feature of a current cycle of sequencing, a signal intensity feature of a preceding cycle of sequencing of the current cycle of sequencing, and a signal intensity feature of a succeeding cycle of sequencing of the current cycle of sequencing;
a base type combination of three cycles of sequencing determined based on the signal intensity feature of the current cycle of sequencing and signal intensity features of two preceding cycles of sequencing of the current cycle of sequencing;
a base type combination of three cycles of sequencing determined based on signal intensity features of three preceding cycles of sequencing of the current cycle of sequencing;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, where the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, where the second parameter characterizes a sum of signal intensities of the four base channels of the current cycle of sequencing.

Optionally, the intensity feature rankings of the signals generated by the current cycle of sequencing in the four base channels A, T/U, C, and G are determined based on sorting, according to intensity magnitudes, raw intensities or corrected intensities of the signals generated by the current cycle of sequencing in the four base channels A, T/U, C, and G.

Optionally, the plurality of groups of sequencing information are determined according to intensity features of signals generated by a plurality of cycles of sequencing, and the plurality of cycles of sequencing are divided into a plurality of segments; each segment of the sequencing includes a preset number of cycles of sequencing, and the preset number of cycles of sequencing within the same segment correspond to the same group of sequencing information.

Optionally, each group of sequencing information, the base calling results, and the preset number of cycles in the table are represented in a data format in integer units, and the data format includes at least one of a data format of a target number of bits, a type data format occupying target bytes, or a binary number format. Optionally, the base calling results include base calling types and/or base sequencing quality scores. Optionally, the base calling results are determined based on each group of sequencing information and a base calling model.

Optionally, the apparatus for base calling includes a model construction unit, and the model construction unit is configured to:
acquire a training sample set, where each training sample in the training sample set is annotated with a feature value and a target value; the feature value is one group of sequencing information in the table, and the target value is a base calling result corresponding to the one group of sequencing information; and
perform machine learning modeling on each sample in the training sample set based on a specific model structure to obtain the base calling model.

Optionally, the specific model structure includes a LightGBM model structure.

Optionally, the query unit includes:
a first determination subunit, configured to determine, in the case where the table includes at least one sub-table, a cycle number range corresponding to the specified cycle of sequencing, where each sub-table in the table has recorded information of a corresponding cycle number range, the recorded information characterizes a plurality of groups of sequencing information under the cycle number range and base calling results forming key-value pairs with each group of sequencing information, and the cycle number range includes a preset number of cycles of sequencing;
a second determination subunit, configured to determine a target sub-table in the table based on the cycle number range; and
a query subunit, configured to perform the information query in the target sub-table based on the at least one group of sequencing information of the specified cycle of sequencing to determine the base calling result of the specified cycle of sequencing.

Correspondingly, in the embodiments of the present application, an apparatus for table generation is further provided. Referring to FIG. 8, the apparatus includes:
a second acquisition unit 401, configured to input each group of sequencing information among a plurality of groups of sequencing information into a base calling model, to obtain base calling results corresponding to each group of sequencing information, where each group of sequencing information is determined based on intensity features of signals generated by a corresponding one cycle of sequencing or a plurality of consecutive cycles of sequencing including the one cycle of sequencing; and
a storage unit 402, configured to store each of the groups of sequencing information and the corresponding base calling results into a preset table in a form of key-value pairs, to obtain a table applied to base calling. Optionally, the intensity features are intensity features obtained after segmentation processing is performed, according to a preset number of segments, on intensity data of the signals generated by the one cycle of sequencing or the plurality of consecutive cycles of sequencing including the one cycle of sequencing, corresponding to each group of sequencing information. The intensity data characterizes a raw intensity or a corrected intensity of a signal; the corrected intensity is an intensity after correction is performed on the raw intensity; optionally, the correction includes at least one of crosstalk correction, phasing/prephasing correction, or spillover correction.

Optionally, each group of sequencing information includes at least one of the following parameters, or a combination thereof:
a base type combination of M+N+1 cycles of sequencing determined based on a signal intensity feature of a current cycle of sequencing, signal intensity features of N preceding cycles of sequencing of the current cycle of sequencing, and signal intensity features of M succeeding cycles of sequencing of the current cycle of sequencing,
where M and N are both integers greater than or equal to 0;
a base type combination of K cycles of sequencing determined based on signal intensity features of K preceding cycles of sequencing of the current cycle of sequencing,
where K is an integer greater than or equal to 1;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, where the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, where the second parameter characterizes a sum of signal intensities of the four base channels of the current cycle of sequencing.

Optionally, each group of sequencing information includes at least one of the following parameters, or a combination thereof:
a base type combination of three cycles of sequencing determined based on a signal intensity feature of a current cycle of sequencing, a signal intensity feature of a preceding cycle of sequencing of the current cycle of sequencing, and a signal intensity feature of a succeeding cycle of sequencing of the current cycle of sequencing;
a base type combination of three cycles of sequencing determined based on the signal intensity feature of the current cycle of sequencing and signal intensity features of two preceding cycles of sequencing of the current cycle of sequencing;
a base type combination of three cycles of sequencing determined based on signal intensity features of three preceding cycles of sequencing of the current cycle of sequencing;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, where the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, where the second parameter characterizes a sum of signal intensities of the four base channels of the current cycle of sequencing.

Optionally, the intensity feature rankings of the signals generated by the current cycle of sequencing in the four base channels A, T/U, C, and G are determined based on sorting, according to intensity magnitudes, raw intensities or corrected intensities of the signals generated by the current cycle of sequencing in the four base channels A, T/U, C, and G.

Optionally, the plurality of groups of sequencing information are determined according to intensity features of signals generated by a plurality of cycles of sequencing, and the plurality of cycles of sequencing are divided into a plurality of segments; each segment of the sequencing includes a preset number of cycles of sequencing, and the preset number of cycles of sequencing within the same segment correspond to the same group of sequencing information.

Optionally, each group of sequencing information, the base calling results, and the preset number of cycles in the table are represented in a data format in integer units, and the data format includes at least one of a data format of a target number of bits, a type data format occupying target bytes, or a binary number format. Optionally, the base calling results include base calling types and/or base sequencing quality scores. Optionally, the base calling results are determined based on each group of sequencing information and a base calling model.

Optionally, the apparatus further includes a construction unit, and the construction unit is configured to: acquire a training sample set, where each training sample in the training sample set is annotated with a feature value and a target value; the feature value is one group of sequencing information in the table, and the target value is a base calling result corresponding to the one group of sequencing information; and perform machine learning modeling on each sample in the training sample set based on a specific model structure to obtain the base calling model.

Optionally, the specific model structure includes a LightGBM model structure.

It should be noted that, in this embodiment, reference may be made to the corresponding disclosure in the foregoing for the specific implementations of each unit and sub-unit, which will not be recited here.

In another embodiment of the present application, a sequencing system is further provided. The sequencing system includes: a memory, configured to store an application program and a table, where the table includes a plurality of groups of sequencing information and base calling results forming key-value pairs with each group of sequencing information; each group of sequencing information is determined based on intensity features of signals generated by a corresponding one cycle of sequencing or a plurality of consecutive cycles of sequencing including the one cycle of sequencing; and
a processor, configured to execute the application program to implement:
acquiring a table; and
acquiring at least one group of sequencing information of a specified cycle of sequencing to perform an information query in the table, so as to determine a base calling result of the specified cycle of sequencing. In another embodiment of the present application, a readable storage medium is further provided. The readable storage medium stores a computer program, and the computer program, when executed by a processor, causes the processor to implement the method for base calling and the method for table generation as described in any one of those above.

It should be noted that, in this embodiment, reference may be made to the corresponding disclosure in the foregoing for the specific implementations of the processor, which will not be recited here.

In the specification, the embodiments are described in a progressive manner. The embodiments focus on their differences from other embodiments, and for the same or similar parts among the embodiments, reference may be made to each other. Since the apparatus disclosed in the embodiments corresponds to the method disclosed in the embodiments, the description of the apparatus is simplified, and reference can be made to the description of the method for the relevant part.

Those skilled in the art would further appreciate that the various exemplary units and algorithm procedures described in the embodiments of the present application may be implemented as electronic hardware, computer software, or a combination of the two, and that the components and procedures of the various embodiments have been described above generally in terms of their functionality in order to clearly illustrate this interchangeability of hardware and software. Whether such functionality is implemented as hardware or software depends upon the specific application and design constraints imposed on the technical solution. Those skilled in the art may implement the described functionality in varying ways for each specific application, but such implementation decisions should not be construed as causing a departure from the scope of the present application.

The procedures of a method or algorithm described in the embodiments of the present application may be implemented directly in hardware, in a software module executed by a processor, or in a combination of the two. The software module may reside in a random access memory (RAM), memory, read-only memory (ROM), electrically programmable ROM, electrically erasable programmable ROM, register, hard disk, removable disk, CD-ROM, or any other form of storage medium known in the art.

The above description of the disclosed embodiments is provided to enable those skilled in the art to implement or use the present application. Various modifications to these embodiments will be readily apparent to those skilled in the art, and the generic principles defined herein may be applied to other embodiments without departing from the spirit or scope of the present application. Thus, the present application is not limited to the embodiments illustrated herein but is in accordance with the broadest scope consistent with the principles and novel features disclosed herein.

## Claims

1. A method for base calling, comprising:
acquiring a table, wherein the table comprises a plurality of groups of sequencing information and corresponding base calling results forming key-value pairs with the respective groups of sequencing information; each of the groups of sequencing information is determined based on intensity features of signals generated by a corresponding one cycle of sequencing or a plurality of consecutive cycles of sequencing including the one cycle of sequencing; and
acquiring at least one group of sequencing information of a specified cycle of sequencing to perform an information query in the table, so as to determine a base calling result of the specified cycle of sequencing.

2. The method for base calling according to claim 1, wherein
the intensity features are intensity features obtained after segmentation processing is performed, according to a preset number of segments, on intensity data of the signals generated by the one cycle of sequencing or the plurality of consecutive cycles of sequencing including the one cycle of sequencing, corresponding to each of the groups of sequencing information,
wherein the intensity data characterizes a raw intensity or a corrected intensity of a signal; and
the corrected intensity is an intensity after correction is performed on the raw intensity;
optionally, the correction comprises at least one of crosstalk correction, phasing/prephasing correction, or spillover correction.

3. The method for base calling according to claim 2, wherein
each of the groups of sequencing information comprises at least one of the following parameters, or a combination thereof:
a base type combination of M+N+1 cycles of sequencing determined based on a signal intensity feature of a current cycle of sequencing, signal intensity features of N preceding cycles of sequencing of the current cycle of sequencing, and signal intensity features of M succeeding cycles of sequencing of the current cycle of sequencing,
wherein M and N are both integers greater than or equal to 0;
a base type combination of K cycles of sequencing determined based on signal intensity features of K preceding cycles of sequencing of the current cycle of sequencing,
wherein K is an integer greater than or equal to 1;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, wherein the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, wherein the second parameter characterizes a sum of signal intensities of the four base channels of the current cycle of sequencing.

4. The method for base calling according to claim 2 or 3, wherein
each of the groups of sequencing information comprises at least one of the following parameters, or a combination thereof:
a base type combination of three cycles of sequencing determined based on a signal intensity feature of a current cycle of sequencing, a signal intensity feature of a preceding cycle of sequencing of the current cycle of sequencing, and a signal intensity feature of a succeeding cycle of sequencing of the current cycle of sequencing;
a base type combination of three cycles of sequencing determined based on the signal intensity feature of the current cycle of sequencing and signal intensity features of two preceding cycles of sequencing of the current cycle of sequencing;
a base type combination of three cycles of sequencing determined based on signal intensity features of three preceding cycles of sequencing of the current cycle of sequencing;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, wherein the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, wherein the second parameter characterizes a sum of signal intensities of the four base channels of the current cycle of sequencing.

5. The method for base calling according to claim 3 or 4, wherein
the intensity feature rankings of the signals generated by the current cycle of sequencing in the four base channels A, T/U, C, and G are determined based on sorting, according to intensity magnitudes, raw intensities or corrected intensities of the signals generated by the current cycle of sequencing in the four base channels A, T/U, C, and G.

6. The method for base calling according to any one of claims 1 to 5, wherein
the plurality of groups of sequencing information are determined according to intensity features of signals generated by a plurality of cycles of sequencing, and the plurality of cycles of sequencing are divided into a plurality of segments; each segment of the sequencing comprises a preset number of cycles of sequencing, and the preset number of cycles of sequencing within a same segment correspond to a same group of sequencing information;
optionally, each of the groups of sequencing information, the base calling results, and the preset number of cycles in the table are represented in a data format in integer units, and the data format comprises at least one of a data format of a target number of bits, a type data format occupying target bytes, or a binary number format;
optionally, the base calling results comprise base calling types and/or base sequencing quality scores; optionally, the base calling results are determined based on each of the groups of sequencing information and a base calling model;
a method for constructing the base calling model comprises:
acquiring a training sample set, wherein each training sample in the training sample set is annotated with a feature value and a target value; the feature value is one group of sequencing information in the table, and
the target value is a base calling result corresponding to the one group of sequencing information; and
performing machine learning modeling on each sample in the training sample set based on a specific model structure to obtain the base calling model;
optionally, the specific model structure comprises a LightGBM model structure.

7. The method for base calling according to claim 6, wherein
acquiring the at least one group of sequencing information of the specified cycle of sequencing to perform the information query in the table, so as to determine the base calling result of the specified cycle of sequencing comprises:
determining, in a case where the table comprises at least one sub-table, a cycle number range corresponding to the specified cycle of sequencing, wherein each sub-table in the table has recorded information of a corresponding cycle number range, the recorded information characterizes a plurality of groups of sequencing information under the cycle number range and corresponding base calling results forming key-value pairs with the respective groups of sequencing information, and the cycle number range comprises a preset number of cycles of sequencing;
determining a target sub-table in the table based on the cycle number range; and
performing the information query in the target sub-table based on the at least one group of sequencing information of the specified cycle of sequencing to determine the base calling result of the specified cycle of sequencing.

8. A method for table generation, comprising:
inputting each group of sequencing information among a plurality of groups of sequencing information into a base calling model, to obtain base calling results corresponding to the respective groups of sequencing information,
wherein each of the groups of sequencing information is determined based on intensity features of signals generated by a corresponding one cycle of sequencing or a plurality of consecutive cycles of sequencing including the one cycle of sequencing; and
storing the groups of sequencing information and the corresponding base calling results into a preset table in a form of key-value pairs, to obtain a table applied to base calling.

9. The method for table generation according to claim 8, wherein
the intensity features are intensity features obtained after segmentation processing is performed, according to a preset number of segments, on intensity data of the signals generated by the one cycle of sequencing or the plurality of consecutive cycles of sequencing including the one cycle of sequencing, corresponding to each of the groups of sequencing information,
wherein the intensity data characterizes a raw intensity or a corrected intensity of a signal; and the corrected intensity is an intensity after correction is performed on the raw intensity;
optionally, the correction comprises at least one of crosstalk correction, phasing/prephasing correction, or spillover correction.

10. The method for table generation according to claim 9, wherein
each of the groups of sequencing information comprises at least one of the following parameters, or a combination thereof:
a base type combination of M+N+1 cycles of sequencing determined based on a signal intensity feature of a current cycle of sequencing, signal intensity features of N preceding cycles of sequencing of the current cycle of sequencing, and signal intensity features of M succeeding cycles of sequencing of the current cycle of sequencing,
wherein M and N are both integers greater than or equal to 0;
a base type combination of K cycles of sequencing determined based on signal intensity features of K preceding cycles of sequencing of the current cycle of sequencing,
wherein K is an integer greater than or equal to 1;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, wherein the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, wherein the second parameter characterizes a sum of signal intensities of the four base channels of the current cycle of sequencing.

11. The method for table generation according to claim 10, wherein
each of the groups of sequencing information comprises at least one of the following parameters, or a combination thereof:
a base type combination of three cycles of sequencing determined based on a signal intensity feature of a current cycle of sequencing, a signal intensity feature of a preceding cycle of sequencing of the current cycle of sequencing, and a signal intensity feature of a succeeding cycle of sequencing of the current cycle of sequencing;
a base type combination of three cycles of sequencing determined based on the signal intensity feature of the current cycle of sequencing and signal intensity features of two preceding cycles of sequencing of the current cycle of sequencing;
a base type combination of three cycles of sequencing determined based on signal intensity features of three preceding cycles of sequencing of the current cycle of sequencing;
intensity feature rankings of signals generated by the current cycle of sequencing in four base channels A, T/U, C, and G;
a first target parameter determined based on a maximum signal intensity value of the current cycle of sequencing, a first parameter, and the preset number of segments, wherein the first parameter characterizes a sum of the maximum signal intensity value of the current cycle of sequencing and a second maximum signal intensity value of the current cycle of sequencing; or
a second target parameter determined based on the maximum signal intensity value of the current cycle of sequencing, a second parameter value, and the preset number of segments, wherein the second parameter characterizes a sum of signal intensities of the four base channels of the current cycle of sequencing.

12. The method for table generation according to claim 10 or 11, wherein
the intensity feature rankings of the signals generated by the current cycle of sequencing in the four base channels A, T/U, C, and G are determined based on sorting, according to intensity magnitudes, raw intensities or corrected intensities of the signals generated by the current cycle of sequencing in the four base channels A, T/U, C, and G.

13. The method for table generation according to any one of claims 8 to 12, wherein
the plurality of groups of sequencing information are determined according to intensity features of signals generated by a plurality of cycles of sequencing, and the plurality of cycles of sequencing are divided into a plurality of segments; each segment of the sequencing comprises a preset number of cycles of sequencing, and the preset number of cycles of sequencing within a same segment correspond to a same group of sequencing information;
optionally, each of the groups of sequencing information, the base calling results, and the preset number of cycles in the table are represented in a data format in integer units, and the data format comprises at least one of a data format of a target number of bits, a type data format occupying target bytes, or a binary number format;
optionally, the base calling results comprise base calling types and/or base sequencing quality scores.

14. The method for table generation according to any one of claims 8 to 13, wherein
a method for constructing the base calling model comprises:
acquiring a training sample set, wherein each training sample in the training sample set is annotated with a feature value and a target value; the feature value is one group of sequencing information in the table, and the target value is a base calling result corresponding to the one group of sequencing information; and
performing machine learning modeling on each sample in the training sample set based on a specific model structure to obtain the base calling model;
optionally, the specific model structure comprises a LightGBM model structure.

15. A table, wherein the table is generated based on the method for table generation according to any one of claims 8 to 14.
